Europäisches Patentamt

European Patent Office

Office européen des brevets

(11) Numéro de publication: **0 273 811 B1**

(12) **FASCICULE DE BREVET EUROPEEN**

(45) Date de publication de fascicule du brevet: **03.08.94**  (51) Int. Cl.⁵: **A61K 39/29**, C12P 21/02

(21) Numéro de dépôt: **87402743.6**

(22) Date de dépôt: **03.12.87**

(54) **Procédé de préparation d'un vaccin contre l'hépatite B.**

(30) Priorité: **10.12.86 FR 8617265**

(43) Date de publication de la demande:
**06.07.88 Bulletin 88/27**

(45) Mention de la délivrance du brevet:
**03.08.94 Bulletin 94/31**

(84) Etats contractants désignés:
**AT BE CH DE ES GB GR IT LI LU NL SE**

(56) Documents cités:
EP-A- 0 168 234     EP-A- 0 199 698
WO-A-85/03876     FR-A- 2 152 800
US-A- 4 088 748     US-A- 4 162 192

BIOLOGICALS, vol. 18, 1990; pp.
345-350&NUM;

(73) Titulaire: **PASTEUR MERIEUX SERUMS ET
VACCINS, Société Anonyme :
58 Avenue Leclerc
F-69007 Lyon(FR)**

(72) Inventeur: **Adamowicz, Philippe Jean
16 Allée des Haras
F-92420 Vaucresson(FR)**
Inventeur: **Mevelec, Marie Noelle
Le Bourg Chevreau
Rivarennes
F-37190 Azay-Le-Rideau(FR)**
Inventeur: **Girard, Marc
6 rue César Franck
F-75015 Paris(FR)**

(74) Mandataire: **Bernasconi, Jean et al
CABINET LEMOINE ET BERNASCONI
13, Boulevard des Batignolles
F-75008 Paris (FR)**

EP 0 273 811 B1

## Description

La présente invention a trait à un procédé de préparation d'un vaccin contre l'hépatite B.

Les difficultés de toutes natures rencontrées pour la préparation industrielle de vaccins contre l'hépatite B par purification de l'antigène HBsAg à partir du sang humain ont conduit tout naturellement à rechercher l'expression de protéines antigéniques à partir de micro-organismes transfectés par des gènes codant du virus de l'hépatite B.

M. Michel et al. ont exprimé des antigènes de l'enveloppe du virus de l'hépatite B à partir de cellules ovariennes de hamster chinois (CHO) (voir référence 1), transfectées par un plasmide portant le gène complet HBsAg. Ces cellules excrètent de façon continue, dans le milieu de culture cellulaire, des particules HBsAg contenant à la fois les protéines pré-S2 et S.

Cependant, lors de cette expression, les particules HBsAg se retrouvent dans le milieu de culture cellulaire contenant des substances indésirables telles que protéines du sérum de veau utilisé pour la croissance cellulaire, protéines et ADN cellulaires, et des particules rétrovirales. Ces particules rétrovirales sont issues de la cellule d'origine avant transfection.

La demande de brevet EP-A-0 168 234 décrit un procédé de purification de HBSAg obtenu par expression dans des cellules recombinantes. Dans ce procédé le surnageant de culture, préalablement concentré, est soumis à un fractionnement en deux étapes par précipitation au sulfate d'amonium, dialysé, puis soumis à une chromatographie sur échangeur d'anions précédant une chromatographie de perméation de gel. En variante ces chromatographies peuvent être remplacées par une adsorption d'immunoaffinité.

Un tel procédé est coûteux car il nécessite le traitement chromatographique ou par immunoaffinité de la préparation présentant encore un pourcentage notable d'impuretés. En outre il serait inutilisable pour garantir l'élimination de particules rétrovirales qui seraient présentes dans le surnageant de culture.

Là présente invention se propose de remédier à ces inconvénients et de fournir un procédé de préparation d'un vaccin contre l'hépatite virale B par expression du gène HBsAg introduit dans des cellules CHO, qui permette de produire, de façon industrielle, un vaccin contre l'hépatite B.

Un autre objectif de l'invention est de fournir un tel procédé de préparation qui permette de réaliser un tel vaccin présentant un degré de pureté extrêmement élevé, notamment en ce qui concerne l'ADN ou des fragments d'ADN cellulaire ainsi qu'en ce qui concerne le rétrovirus contaminant les cellules CHO.

Un autre objectif de l'invention est de réaliser un tel vaccin qui présente un pouvoir protecteur particulièrement élevé.

L'invention a pour objet un procédé de préparation d'un vaccin recombiné contre l'hépatite B comprenant à la fois les protéines pré-S2 et S de l'antigène de surface du virus de l'hépatite B, dans lequel on produit des particules antigéniques de surface de l'hépatite B par expression à partir d'une culture de cellules CHO transfectées par un plasmide portant le gène HBsAg de façon à libérer les particules de surface antigéniques dans le milieu de culture, caractérisé en ce que

- l'on récupère le milieu surnageant de culture d'au moins une culture, de préférence effectuée avec une faible teneur en sérum animal,
- on effectue une filtration stérilisante du surnageant,
- on procède à une concentration, de préférence d'environ cinquante fois, de préférence par ultrafiltration,
- on effectue une précipitation du concentrat à l'aide d'un agent précipitant non dégradant dans des conditions précipitant les classes lourdes d'ADN, ladite précipitation entraînant également les particules rétrovirales, et des protéines,
- on effectue une nouvelle concentration de la préparation antigénique, à l'aide d'un agent précipitant non dégradant dans des conditions précipitant les particules antigéniques de surface HBsAg, puis on reprend le précipité dans un faible volume,
- on effectue une centrifugation zonale de vélocité dans un gradient de densité non chaotropique pour les rétrovirus de sorte que leur intégrité soit conservée pour permettre leur séparation des particules HBsAg, suivant leurs taille et densité, de préférence en obtenant aussi une discrimination entre les particules antigéniques HBsAg d'une part, une séparation entre HBsAg, ADN et protéines, d'autre part,
- on effectue une centrifugation zonale isopycnique de flottation éliminant les acides nucléiques légers et lourds, et les protéines, et
- on effectue une chromatographie sur échangeur d'anions pour adsorber les traces d'acides nucléiques et de protéines non HBsAg restantes.

Par agents précipitants non dégradants, on entend des agents qui, bien sûr, ne dégradent pas les antigènes HBsAg, et notamment ceux de type S et pré S, mais aussi qui n'ont pas d'effet chaotropique ou dissociant pour les particules rétrovirales. L'agent préféré est le polyéthylène glycol (PEG). Parmi d'autres agents, on peut citer notamment le sulfate d'ammonium.

Les étapes de purification ne dénaturent donc pas les particules HBsAg, et notamment ne dénaturent pas les protéines pré S qui sont les plus exposées. Parmi les agents précipitants convenables, on préfère le polyéthylène glycol (PEG) en choisissant notamment un poids moléculaire de l'ordre de 4.000 à 20.000, ce qui permet dans des conditions convenables, telles que notamment une concentration en PEG de l'ordre de 5%, de précipiter les particules rétrovirales en permettant également de précipiter notablement les classes lourdes d'ADN tout en conservant dans le surnageant du précipité les particules HBsAg. Celles-ci peuvent être récupérées dans un volume très faible, de préférence après les avoir précipitées par addition de PEG à concentration plus forte.

Le gradient de densité dans lequel on effectue la centrifugation zonale de vélocité est également prévu pour ne pas altérer l'antigène HBsAg, antigénicités S et pré S incluses. De plus, ce gradient est prévu pour ne pas avoir d'effet chaotropique sur les particules rétrovirales de sorte que leur intégrité soit conservée. De préférence, ce gradient, de faible force ionique, est un gradient de saccharose. En variante, on peut également utiliser d'autres gradients de densité, notamment le glycérol.

La centrifugation zonale de vélocité qui est ainsi effectuée dans un tel gradient de densité permet ainsi d'éliminer, de façon extrêmement efficace, les particules rétrovirales. En même temps, on peut obtenir une certaine purification en ADN, notamment en chaînes lourdes, et en protéines.

La centrifugation zonale isopycnique de flottation sur un gradient salin de densité, tel que KBr, est ainsi effectuée sur une préparation pratiquement totalement dépourvue de particules rétrovirales. Cependant, si des particules rétrovirales contaminaient l'échantillon à purifier, l'effet chaotropique du sel constituant le gradient de densité, détruirait la structure virale et libérerait le génome viral à ARN dans la zone dense du gradient où il stationnerait, permettant ainsi sa séparation de l'antigène HBsAg.

Cette étape permet une élimination de la presque totalité des protéines ainsi que des traces d'ADN léger ou lourd subsistantes.

Enfin l'étape de chromatographie d'échange d'anions permet d'adsorber les éventuelles traces d'acides nucléiques, aussi bien ADN qu'ARN encore restantes, et de protéines.

D'autres avantages et caractéristiques de l'invention apparaîtront à la lecture de la description suivante, faite à titre d'exemple non limitatif.

1. PREPARATION DE LA CULTURE CELLULAIRE.

Un clone de lignée cellulaire CHO transfectée par un plasmide recombinant (demande de brevet FR-A-84.03564 publiée sous le n° 2.560.890) comprenant le gène du virus de l'hépatite B codant pour l'antigène HBs (régions pré-S et S), est démultiplié à partir d'une ampoule de banque cellulaire, en unités de fermentation de volumes croissants. La dernière démultiplication est réalisée en fermenteur de 300 litres, les cellules se propageant sur des microbilles en suspension dans le milieu de culture. Pour ces étapes de démultiplication, le milieu de croissance contient 4 à 10 % de SV (sérum de veau), préférablement 5 %.

2. CULTURES, RECOLTES ET CONCENTRATION.

La première récolte est effectuée au bout de 3 à 4 jours de culture dans le fermenteur de 300 litres : après décantation des billes supportant les cellules, 250 litres de milieu surnageant sont soutirés et remplacés par un volume équivalent de milieu neuf contenant 0,2 à 4 % de SV, préférablement 1 à 2 %. La culture cellulaire est poursuivie dans le fermenteur pendant 3 à 4 jours au bout desquels une nouvelle récolte de 250 litres est effectuée. On peut opérer ainsi jusqu'à 10 récoltes successives, qui sont conservées à +4°C en vue d'être purifiées. En régle générale, le première récolte, la récolte n° 7 et les suivantes ont un faible titre en antigène HBs, de sorte qu'elles ne sont pas retenues pour les étapes de purification.

Dans l'exemple présent, les récoltes n° 2 à 6 ont été conservées pour être purifiées et contenaient 1 % de SV.

Après mélange des récoltes, la préparation d'un volume de 1250 litres est filtrée sur membrane 0,2 $\mu$m, puis concentrée par ultrafiltration d'environ 50 fois, sur membrane de point de coupure 100.000 daltons, ce qui donne la récolte concentrée R.

## 3. PRECIPITATIONS FRACTIONNEES.

La récolte concentrée R est soumise à un fractionnement par précipitations sélectives, dans un premier temps des composants de poids moléculaires élevés, particulièrement classes lourdes d'ADN et particules rétrovirales, l'antigène HBs restant en suspension, et dans un deuxième temps de l'antigène HBs. L'agent précipitant est choisi de manière telle que les particules rétrovirales conservent leur taille et leur densité d'origine d'une part, et que d'autre part l'antigénicité des particules HBs ne soit pas altérée. L'agent précipitant préféré est le polyéthylène glycol, préférablement de PM 6.000.

On utilise une concentration de 5 % en PEG 6.000 pour la première précipitation et le précipité obtenu est éliminé par centrifugation. Le surnageant S est additionné de PEG 6.000 pour une concentration finale de 9,5 % p/v; après centrifugation, le précipité est dissous totalement dans un volume égal environ au 625ème du volume de récoltes traité, soit 2 litres, environ, de préparation antigénique EC.

A ce stade, les performances d'élimination de l'ADN cellulaire et des protéines non-HBsAg sont indiquées dans le tableau suivant :

| | Volume en l | ADN | | | Protéines non-HBsAg | | |
|---|---|---|---|---|---|---|---|
| | | µg/ml | µg totaux | % | µg/ml | µg totaux | % |
| Mélange | 1250 | $520 \times 10^{-3}$ | 650000 | 100 | 740 | $925 \times 10^6$ | 100 |
| Récolt. R | 25,15 | $41 \times 10^{-3}$ | 1031 | 0,16 | 14860 | $374 \times 10^6$ | 40,4 |
| Surnag. S | 27,84 | $1,3 \times 10^{-3}$ | 36,25 | 0,0056 | 8666 | $241 \times 10^6$ | 26,0 |
| EC | 2,03 | $7,67 \times 10^{-3}$ | 15,6 | 0,0024 | 11850 | $24,06 \times 10^6$ | 2,6 |

Tandis que le taux de récupération de l'antigène HBs était de 80 %, des facteurs d'élimination de 42.000 pour l'ADN et de 38,5 pour les protéines ont été constatés.

L'activité reverse transcriptase reflétant le titre en rétrovirus, est non détectable dans les récoltes et parfois légèrement positive dans le récolte concentrée de 50 fois et, dans tous les cas, elle est négative dans le surnageant après traitement au PEG 5% p/v.

Le pouvoir d'élimination d'un rétrovirus par cette étape de traitement au PEG 5 % p/v a été mesuré en supplémentant une récolte concentrée avec du MuLV (Murine Leukemia Virus) : un facteur d'élimination de 7 a été obtenu.

## 4. CENTRIFUGATION ZONALE DE VELOCITE.

Un gradient de saccharose (0 - 40 %) est formé dans un rotor K2-Electronucleonics (référence 2) ; lorsque la vitesse de rotation atteint 35.000 t/min, le rotor est alimenté à un débit continu de 2 l/h d'abord avec 500 ml d'échantillon EC à purifier (concentrat), puis pendant les 30 min suivantes avec un tampon phosphate 65 mM pH 6,8. Après arrêt du rotor, le contenu du rotor est fractionné et les fractions titrées pour leur contenu en antigène HBs. Les fractions riches en HBsAg sont retrouvées dans les couches les moins denses du gradient, contenant moins de 25 % p/v de saccharose ; elles sont mélangées avec celles issues du traitement, dans des conditions identiques, des autres fractions de 500 ml de concentrat.

Le mélange de ces fractions est dialysé pour éliminer le saccharose et ensuite concentré par ultrafiltration jusqu'à un volume de 0,36 l. On obtient une préparation Z10. Le rendement en HBsAg est de 80 %. Les performances d'élimination de l'ADN et des protéines non HBsAg ont été :

4

EP 0 273 811 B1

| | Volume | ADN | | | Protéines non- HBsAg | | |
|---|---|---|---|---|---|---|---|
| | en l | $\mu$g/ml | $\mu$g totaux | % | $\mu$g/ml | $\mu$g totaux | % |
| EC | 2,03 | $7,67 \times 10^{-3}$ | 15,6 | 0,0024 | 11850 | $24,06 \times 10^6$ | 2,6 |
| Z10 | 0,36 | $16,2 \times 10^{-3}$ | 5,825 | 0,00093 | 17330 | $6,24 \times 10^6$ | 0,67 |

Bien que donnant des résultats appréciables d'élimination de l'ADN et des protéines non-HBsAg, cette centrifugation de vélocité est surtout intéressante pour son pouvoir de discrimination entre l'antigène et les particules rétrovirales.

En effet, lorsque le concentrat est délibérément contaminé par un rétrovirus murin (Murine Leukemia Virus), puis soumis à cette centrifugation dans les conditions précitées, on retrouve les particules rétrovirales titrées en reverse transciptase dans les couches denses du gradient avec un pic d'activité enzymatique vers 33-35 % de saccharose.

Les particules rétrovirales ont rejoint leur zone isodense du gradient, en parcourant la plus grande partie du gradient, tandis que dans le même temps les particules HBsAg, de constante de sédimentation plus faible (40 S) n'ont pu parcourir qu'un faible chemin dns le gradient. Leur séparation est assurée par un temps court de centrifugation.

L'activité reverse transcriptase mesurée dans le mélange des fractions riches en HBsAg représente 0,013 % de celle retrouvée dans le pic rétroviral, soit un facteur d'élimination de 77.

## 5. CENTRIFUGATION ISOPYCNIQUE DE FLOTTATION.

Le rotor est un rotor zonal de type Ti15-Beckman vendu par la société Beckman, Palo Alto, Californie, USA, de capacité totale 1700 ml environ (référence 3). Un gradient de densité est constitué en introduisant par la périphérie du rotor des volumes successifs de solution de bromure de potassium (KBr) de manière à constituer un gradient de densités comprises entre 1,17 et 1,35. L'une des couches introduites est constituée de l'échantillon additionné de KBr pour que la densité de la couche soit égale à 1,25, de sorte qu'en début de centrifugation, toutes les impuretés et l'antigène se retrouvent dans la partie dense du gradient.

Après centrifugation à 31.000 t/min pendant 20 h, et après fractionnement du contenu du rotor, les particules HBsAg sont retrouvées dans leur zone isodense (d 1,22 - 1,23) tandis que la presque totalité des autres protéines et de l'ADN est retrouvée dans les couches denses du gradient. Le mélange des fractions riches en HBsAg est dialysé contre du tampon phosphate 65 mM pH 6,8 pour éliminer le KBr et équilibrer l'échantillon dans le tampon qui sera utilisé pour la chromatographie. On obtient la préparation Z20.

Tandis que le rendement en HBsAg dans cette centrifugation est proche de 100 %, des facteurs très importants d'élimination d'ADN et protéines non-HBsAg sont obtenus :

| | Volume en l | ADN | | | Protéines non-HBsAg | | |
|---|---|---|---|---|---|---|---|
| | | $\mu$g/ml | $\mu$g totaux | % | $\mu$g/ml | $\mu$g totaux | % |
| $Z_{10}$ | 0,360 | 0,0162 | 5,825 | $9,3 \times 10^{-4}$ | 17330 | $6,24 \times 10^{6}$ | 0,67 |
| $Z_{20}$ | 0,458 | $36 \times 10^{-6}$ | 0,0166 | $2,7 \times 10^{-6}$ | 234 | $107 \times 10^{3}$ | 0,011 |

Ils sont respectivement de 350 pour l'ADN et de 58 pour les protéines non-HBsAg.

Un facteur d'élimination de rétrovirus n'a pu être calculé dans cette étape, car l'activité reverse transcriptase est totalement détruite en milieu KBr. Cette inactivation est due vraisemblablement à l'effet chaotropique du sel sur le virus. Il est intéressant de noter que cet effet chaotropique devrait libérer le génome viral (ARN) dans le milieu qui se retrouverait après centrifugation dans les couches denses du gradient comme l'ADN cellulaire, et ainsi séparé du pic d'antigène.

6. CHROMATOGRAPHIE D'ECHANGE D'ANIONS.

La préparation antigénique Z20 dialysée après la centrifugation isopycnique est passée sur échangeur d'anions, préférablement sur DE 52-cellulose Whatman® préalablement équilibrée en tampon phosphate 65 mM pH 6,8. Dans ces conditions, l'antigène HBs n'est pas adsorbé sur l'échangeur, tandis que les traces d'ADN résiduelles et les protéines contaminantes se fixent sur l'échangeur :

| | Volume en l | ADN | | | Protéines non-HBsAg | | |
|---|---|---|---|---|---|---|---|
| | | $\mu$g/ml | $\mu$g totaux | % | $\mu$g/ml | $\mu$g totaux | % |
| $Z_{20}$ | 0,458 | $36 \times 10^{-6}$ | 0,0166 | $2,7 \times 10^{-6}$ | 234 | $107 \times 10^{3}$ | 0,011 |
| après chromato | 0,600 | $< 12,5 \times 10^{-6}$ | $< 0,0075$ | $< 1,2 \times 10^{-6}$ | 7 | 4200 | $4,3 \times 10^{-4}$ |

Un facteur important d'élimination des protéines non-HBsAg est obtenu, de 25 fois, tandis que l'ADN n'est plus décelable par la technique dot-blot après hybridation avec une sonde marquée au P32 (limite de sensibilité = 12,5 pg/ml).

Pour mesurer le facteur d'élimination de l'ADN, une préparation Z20 a été additionnée d'une quantité connue d'ADN et chromatographiée sur l'échangeur d'anions. Le taux d'ADN retrouvé dans l'effluent de la colonne était 1.000 à 10.000 fois inférieur au titre initial.

La préparation antigénique ainsi obtenue après purification présente les caractéristiques suivantes :

| . Protéine HBsAg | 1 $\mu$g |
|---|---|
| . Protéine non HBsAg | ~ 50 ng |
| . ADN | ~ $10^{-5}$ pg |

Après électrophorèse sur gel de polycrylamide en milieu dissociant, les polypeptides colorés par le nitrate d'argent se distribuent en 3 bandes majeures :

. $P_{22}$ de poids moléculaire apparent 22.000 daltons

. $P_{26}$ de poids moléculaire apparent 26.000 daltons

. $P_{34}$ de poids moléculaire apparent 34.000 daltons

qui sont reconnus tous les trois par immunoempreinte avec un anticorps monoclonal anti-S et dont seul le polypeptide $P_{34}$ est reconnu par immunoempreinte avec un anticorps monoclonal anti-pré S2.

## 7. PREPARATION DE VACCINS ET IMMUNOGENICITE.

La préparation chromatographiée est filtrée sur membrane stérilisante, puis elle est chauffée à +60°C pendant 60 min et, après refroidissement, elle est additionnée de formaldéhyde (100 $\mu$g/ml) et incubée pendant 48 h à +30°C. Cette préparation antigénique est diluée en tampon physiologique et additionnée d'un adjuvant tel que l'hydroxyde d'aluminium pour obtenir des vaccins.

Des doses différentes d'antigènes HBs ont été injectées par voie sous-cutanée à des cobayes, et les réponses anticorps (anti-S et anti-pré S2) ont été mesurées et comparées à celles obtenues chez des animaux injectés avec des doses égales d'antigène dérivé du plasma humain (vaccin Hévac B - Pasteur Vaccins - MARNES-LA-COQUETTE - FRANCE).

| ANIMAUX REPONDEURS / ANIMAUX INJECTES | | | |
|---|---|---|---|
| Anti-S réponse | | Anti-pré S2 réponse | |
| HEVAC B | CHO | HEVAC B | CHO |
| 10 $\mu$g | | | |
| 5/10 | 8/10 | 7/10 | 10/10 |
| 5 $\mu$g | | | |
| 4/9 | 4/10 | 2/10 | 10/10 |
| 2,5 $\mu$g | | | |
| 1/10 | 0/10 | 2/10 | 5/10 |
| Puissance | | | |
| 1,0 | 1,2 | 1,0 | 3,0 |

Les anticorps anti-S, généralement appelés anti-HBsAg, étaient mesurés par la technique Ausab-Abbott® (Abbot Laboratories, USA) et les anticorps anti-pré S2 étaient mesurés par méthode immunoenzymatique en utilisant un peptide synthétique de 31 aminoacides sur la phase solide, comprenant l'épitope pré S2, pour capter les anticorps qui étaient reconnus dans un deuxième temps par un anticorps anti-IgG conjugué à la peroxydase ; après addition du substrat ($H_2O_2$) et d'un chromogène (orthophénylène diamine), la coloration développée était mesurée à 492 nm, dont l'intensité est proportionnelle à la quantité d'anticorps captés.

Comme l'indiquent les résultats du tableau, une réponse équivalente en anti-S est obtenue avec les deux types de vaccin hépatite B, tandis qu'une plus forte réponse anti-pré S2 est observée avec le vaccin recombiné (CHO).

Références.

1. M. Michel, (1984) Proc. Natl. Acad. Sci. USA 81,7708-7712.
2. Progress In Separation and Purification, édité par Perry and Van Oss,Copyright 1971 by John Wiley & Sons Inc, USA.
3. A. Fritsch, Les Centrifugations Préparatives en Gradient de densité, 2ème édition.

## Revendications

1. Procédé de préparation d'un vaccin recombiné contre l'hépatite B comprenant à la fois les protéines pré-S2 et S de l'antigène de surface du virus de l'hépatite B, dans lequel on produit des particules antigéniques de surface de l'hépatite B par expression à partir d'une culture de cellules CHO (Chinese hamster ovary cells) tranfectées par un plasmide portant le gène HBsAg de façon à libérer les particules de surface antigéniques dans le milieu de culture, caractérisé en ce que
   - on récupère le milieu surnageant de culture d'au moins une culture, notamment en un milieu à faible teneur en sérum animal,
   - on effectue une filtration stérilisante du surnageant,
   - on procède à une concentration du surnageant,
   - on effectue une précipitation du concentrat à l'aide d'un agent précipitant non dégradant dans des conditions précipitant les classe lourdes d'ADN, les particules rétrovirales, ainsi que des protéines,
   - on effectue une nouvelle concentration à l'aide d'un agent précipitant non dégradant dans des conditions précipitant les particules antigéniques de surface HBsAg, puis on reprend le précipité dans un faible volume,
   - on effectue une centrifugation zonale de vélocité dans un gradient non chaotropique pour les particules rétrovirales et choisi pour permettre leur séparation d'avec les particules HBsAg d'après leurs taille et densité,
   - on effectue une centrifugation zonale isopycnique de flottation éliminant les acides nucléiques légers et lourds et des protéines, et,
   - on effectue une chromatographie en milieu échangeur d'anions pour adsorber les traces d'acides nucléiques restantes et les protéines non-HBsAg restantes.

2. Procédé selon la revendication 1, caractérisé en ce que l'on effectue les précipitations au polyéthylène glycol, notamment PEG 6000.

3. Procédé selon la revendication 2, caractérisé en ce que l'on effectue la première précipitation à une concentration de PEG de l'ordre de 5 % p/v.

4. Procédé selon l'une des revendications 1 à 3, caractérisé en ce que l'on effectue la seconde précipitation au PEG, notamment PEG 6000 à 9,5 % p/v.

5. Procédé selon l'une quelconque des revendications 1 à 4, caractérisé en ce que l'on effectue la centrifugation zonale de vélocité en gradient saccharose.

6. Procédé selon la revendication 5, caractérisé en ce que l'on effectue la centrifugation dans un rotor alimenté en continu.

7. Procédé selon l'une des revendications 5 et 6, caractérisé en ce que l'on sélectionne les fractions ayant un taux de saccharose inférieur à 25 % p/v, les autres fractions étant rejetées.

8. Procédé selon l'une quelconque des revendications 1 à 7, caractérisé en ce que l'on effectue la centrifugation zonale de flottation en gradient KBr.

**9.** Procédé selon la revendication 8, caractérisé en ce que l'on recueille les fractions du pic de densité 1,22-1,23.

**10.** Procédé selon l'une quelconque des revendications 1 à 9, caractérisé en ce que l'on effectue la chromatographie sur DE 52 cellulose.

**Claims**

**1.** A method for preparing a recombinant vaccine against hepatitis B comprising both pre-S2 ans S proteins from the surface antigen of the hepatitis B virus, in which surface antigenic particles of hepatitis B are produced by expression from a culture of CHO cells (Chinese hamster ovary cells) transfected by a plasmid carrying the HBsAg gene so as to release the antigenic surface particles in the culture medium, characterized in that
- the supernatant culture medium is recovered from at least one culture, particularly in a medium with a low animal serum content,
- the supernatant is subjected to sterilizing filtration,
- the supernatant is concentrated,
- the concentrate is precipitated by means of a non degrading precipitating agent under conditions precipitating the heavy DNA classes, the retroviral particles and proteins,
- a new concentration is carried out by means of a non degrading precipitating agent under conditions precipitating the surface antigenic HBsAg particles, then the precipitant is redissolved in a small volume,
- a zonal rate centrifugation is carried out in a chaotropic density gradient for the retroviral particles and chosen so as to allow separation thereof from the HBsAg particles, depending on their size and density,
- an isopycnic zonal centrifugation of flotation type is carried out eliminating the light and heavy nucleic acids and the proteins, and
- chromatography is effected on an anion exchange medium so as to adsorb the remaining traces of nucleic acids and remaining non HBsAg proteins.

**2.** Method according to claim 1, characterized in that the precipitations are carried out with polyethylene glycol, particularly PEG 6000.

**3.** Method according to claim 2, characterized in that the first precipitation is carried out with a PEG concentration of the order of 5 % p/v.

**4.** Method according to one of the claims 1 to 3, characterized in that the second precipitation is carried out with PEG, particularly PEG 6000 at 9.5 % p/v.

**5.** Method according to any one of claim 1 to 4, characterized in that the rate zonal centrifugation is carried out in a sucrose gradient.

**6.** Method according to claim 5, characterized in that the centrifugation is carried out in a rotor alimented in a continuous manner.

**7.** Method according to one of claim 5 and 6, characterized in that the fractions having a sucrose rate less than 25 % p/v are selected, the other fractions being rejected.

**8.** Method according to any one of claim 1 to 7, characterized in that the zonal flotation type centrifugation is carried out in a KBr gradient.

**9.** Method according to claim 8, characterized in that the fractions of the density peak 1.22 -1.23 are collected.

**10.** Method according to any one of claims 1 to 9, characterized in that the chromatography is carried out on DE 52 cellulose.

**Patentansprüche**

1. Verfahren zur Herstellung eines rekombinierten Impfstoffes gegen Hepatitis B, der gleichzeitig die Proteine Prä-S2 und S des Oberflächenantigens des Hepatitis B-Virus enthält, in dem man antigene Oberflächenteilchen von Hepatitis B ausgehend von einer Kultur von CHO-Zellen (Ovarialzellen des chinesischen Hamsters), die mit einem das HBsAg-Gen tragenden Plasmid transfiziert sind, durch Expression derart erzeugt, daß die antigenen Oberflächenteilchen im Kulturmedium freigesetzt werden, dadurch gekennzeichnet, daß
   - man das überstehende Kulturmedium mindestens einer Kultur, insbesondere in einem Medium mit einem schwachen Tierserumgehalt, gewinnt,
   - man eine Sterilfiltration des Überstands vornimmt,
   - man eine Konzentration des Überstands vornimmt,
   - man eine Fällung des Konzentrats vornimmt mit Hilfe eines Fällungsmittels, das nicht abbauend ist, unter Bedingungen, die die schweren Klassen von DNA, die retroviralen Teilchen sowie Proteine fällen,
   - man eine erneute Konzentration vornimmt mit Hilfe eines Fällungsmittels, das nicht abbauend ist, unter Bedingungen, die die antigenen HBsAg-Oberflächenteilchen fällen, dann den Niederschlag in einem geringen Volumen aufnimmt,
   - man eine Geschwindigkeits-Zonalzentrifugation in einem Gradienten vornimmt, der für die retroviralen Teilchen nicht chaotrop ist und der so ausgewählt ist, daß er deren Trennung von HBsAg-Teilchen nach deren Größe und Dichte gestattet,
   - man eine isopyknische Flotations-Zonalzentrifugation vornimmt, die die leichten und schweren Nukleinsäuren und Proteine entfernt, und
   - man eine Chromatographie in einem Anionenaustauschmedium vornimmt, um die verbleibenden Spuren von Nukleinsäuren und die verbleibenden Nicht-HBsAg-Proteine zu adsorbieren.

2. Verfahren nach Anspruch 1, dadurch gekennzeichnet, daß man die Fällungen mit Polyethylenglykol, insbesondere PEG 6000, bewirkt.

3. Verfahren nach Anspruch 2, dadurch gekennzeichnet, daß man die erste Fällung mit einer Konzentration an PEG in der Größenordnung von 5% Gew./Vol. bewirkt.

4. Verfahren nach einem der Ansprüche 1 bis 3, dadurch gekennzeichnet, daß man die zweite Fällung mit PEG, insbesondere PEG 6000, zu 9,5% Gew./Vol. bewirkt.

5. Verfahren nach irgendeinem der Ansprüche 1 bis 4, dadurch gekennzeichnet, daß man die Geschwindigkeits-Zonalzentrifugation in einem Saccharose-Gradienten vornimmt.

6. Verfahren nach Anspruch 5, dadurch gekennzeichnet, daß man die Zentrifugation in einem Rotor vornimmt, den man kontinuierlich beschickt.

7. Verfahren nach einem der Ansprüche 5 und 6, dadurch gekennzeichnet, daß man die Fraktionen mit einem Saccharose-Gehalt unterhalb von 25% Gew./Vol. auswählt, wobei die anderen Fraktionen verworfen werden.

8. Verfahren nach irgendeinem der Ansprüche 1 bis 7, dadurch gekennzeichnet, daß man die Flotations-Zonalzentrifugation in einem KBr-Gradienten vornimmt.

9. Verfahren nach Anspruch 8, dadurch gekennzeichnet, daß man die Fraktionen der Bande der Dichte 1,22-1,23 sammelt.

10. Verfahren nach irgendeinem der Ansprüche 1 bis 9, dadurch gekennzeichnet, daß man die Chromatographie auf DE 52-Cellulose vornimmt.